# FASCICULE DE BREVET EUROPEEN

(11) **EP 1 837 005 B1**
(45) Date de publication et mention de la délivrance du brevet: **16.12.2009**
(21) Numéro de dépôt: 07104406.9
(22) Date de dépôt: 19.03.2007
(51) Int. Cl.: A61J 1/05, A61M 39/18

(54) **CONNECTEUR MEDICAL DE TYPE LUER ET SET DE TRANSFERT COMPORTANT UN TEL CONNECTEUR**
Luer Anschluss und Flüssigkeitstransferset mit diesem Anschluss
Medical Luer connector and transfer set having this connector

(30) Priorité: 24.03.2006 FR 0651030
(43) Date de publication de la demande: 26.09.2007
(73) Titulaire: Technoflex, 64210 Bidart (FR)
(72) Inventeur: Capitaine, Francois, 64600, ANGLET (FR); Durand, Francois, 64100, BAYONNE (FR)
(74) Mandataire: Schmit, Christian Norbert Marie

(56) Documents cités:
- EP-A1- 0 499 481
- EP-A1- 1 378 223
- WO-A-98/48765
- WO-A-02/056946
- WO-A-2004/091472
- WO-A2-03/077826
- DE-C1- 10 146 007

## Description

La présente invention concerne un connecteur luer, un connecteur médical et un set de transfert comportant un tel connecteur luer ou connecteur médical pour l'administration à des fins médicales de substances liquides.

Ce set de transfert est notamment destiné à la reconstitution d'un principe actif, tel qu'une poudre médicamenteuse pour le traitement du cancer, contenu dans un flacon à partir d'un constituant liquide.

La reconstitution est généralement réalisée par un opérateur avant administration du médicament au patient. Cette reconstitution est obtenue, à titre d'exemple, par introduction dans le flacon contenant le principe actif d'une solution à diluer renfermée dans un récipient tel qu'une poche souple ou un flacon.

Pour éviter d'éventuelles erreurs humaines lors d'une telle manipulation, on utilise généralement des ensembles de reconstitution constitués notamment d'un connecteur destiné à venir se fixer au flacon contenant le principe actif.

Ce connecteur peut comporter une cloche permettant sa fixation sur le col d'un flacon par clipsage, la cloche enserrant alors au moins une portion de celui-ci.

Toutefois, ce connecteur de l'état de l'art est particulièrement complexe car il est typiquement constitué d'un ensemble de diverses pièces venant s'emboîter les unes dans les autres.

On constate alors que quelque soit le soin apporté à la constitution du connecteur, il existe entre ces différentes pièces, des espaces susceptibles de piéger des quantités du fluide contenant le principe actif que l'on cherche à administrer au patient.

De plus, les connecteurs de l'état de l'art sont généralement de grandes dimensions. Par conséquent, ces pertes peuvent encore être accrues par la présence de surfaces de contact importantes entre le connecteur et le fluide contenant le principe actif, lesquelles sont susceptibles de retenir une certaine quantité de ce fluide.

Ces dispositifs de reconstitution ne permettent donc pas un transfert optimal du fluide contenant le principe actif après reconstitution.

Or les poudres médicamenteuses pour le traitement du cancer sont d'un coût très élevé. Elles sont, de plus, prédosées pour correspondre exactement à la dose requise pour une phase de traitement particulière du patient.

Ces pertes peuvent entraîner une administration insuffisante du traitement au patient avec les conséquences qui peuvent en découler sur l'état de santé de ce dernier. Ce qui peut éventuellement s'avérer plus grave, selon la pathologie, le patient peut recevoir des doses qui sont variables d'une phase de traitement à une autre.

Par ailleurs, ces connecteurs ne peuvent venir s'adapter sur n'importe quel flacon existant. Ils sont destinés à être connectés à des flacons ayant des formes prédéterminées puisque la cloche vient recouvrir au moins partiellement le corps du flacon. Il est donc nécessaire de disposer d'un ensemble de connecteurs pour réaliser des connexions avec des flacons différents.

Ces connecteurs de l'état de l'art permettent par ailleurs de réaliser des pré-connexions sans communication pour conditionner des ensembles connecteur/flacon prêts à l'emploi. Ces ensembles ainsi réalisés peuvent alors être stockés en vue d'une utilisation ultérieure.

Cependant, on constate qu'il est difficile de contrôler, avant utilisation, l'état de ces connecteurs pour vérifier si la stérilité de l'ensemble mis en oeuvre est encore entière.

Il est, par ailleurs, connu que le perçage du bouchon d'un flacon contenant un principe actif, tel qu'une poudre, génère une légère aspiration de l'air extérieur au flacon au travers du connecteur. Cette aspiration peut être à l'origine avec les connecteurs de l'état de l'art d'une contamination du contenu du flacon.

De même, l'accouplement d'un connecteur luer mâle et d'un connecteur luer femelle tel qu'obtenu par le vissage d'une virole sur un filetage nécessite une attention particulière de l'opérateur lors de sa réalisation pour s'assurer de l'étanchéité de cette liaison.

L'opérateur doit notamment s'assurer d'un complet vissage de la virole du premier connecteur sur le filetage de l'autre connecteur. Une erreur de manipulation, par exemple un vissage insuffisant, peut résulter en une contamination du flacon.

Il serait donc intéressant de renforcer la sécurité du connecteur pour éviter que le contenu du flacon ne puisse éventuellement être exposé à des contaminants lors de sa connexion par un opérateur à l'élément de connexion d'une poche à soluté.

On connaît par ailleurs un set de transfert pour l'administration d'un mélange de liquides à des fins médicales de la présente demandeuse (WO 02/056946). Ce mélange de liquides est notamment obtenu à partir d'une poche souple dite principale contenant un premier liquide et d'au moins une poche souple dite auxiliaire contenant un second liquide, raccordable à la poche principale à l'aide d'une paire de connecteurs de type luer respectivement mâle et femelle.

Chacun de ces connecteurs comporte, à une extrémité, une section frangible engagée dans l'extrémité d'une tubulure reliée à l'une des poches et, à l'autre extrémité, un moyen d'accouplement à l'autre connecteur, pourvu de moyens de blocage en position accouplée desdits connecteurs.

Ce set de transfert permet avantageusement de proposer des ensembles d'administration prêts à l'emploi. Ce set de transfert comprend alors une poche principale par exemple de diluant pré-connectée, c'est à dire raccordée par une liaison tubulaire mais non communicante, à une poche dosée ou en parallèle à plusieurs poches dosées de principes actifs ou de nutriments, et dont la communication avec la poche de diluant peut être réalisée, in situ, à tout moment et instantanément par rupture des sections frangibles des luers de la communication à établir.

Ce set de transfert qui donne des résultats très satisfaisants peut néanmoins être amélioré notamment pour son utilisation avec un flacon contenant un principe actif.

Le connecteur luer destiné à être relié au flacon nécessite en effet l'utilisation d'un connecteur adapté sur le flacon pour son assemblage. Le connecteur doit disposer d'une tubulure à son extrémité pour permettre l'introduction de l'extrémité d'un connecteur luer. Le set de transfert devenant complexe, des pertes de principe actif et/ou une contamination du contenu du flacon sont en conséquence possibles lors de la reconstitution.

La présente invention vise à pallier ces divers inconvénients en proposant un connecteur luer et un connecteur médical comportant un tel connecteur qui soient particulièrement simples dans leur conception et dans leur mode opératoire, compacts, économiques et permettant de limiter au maximum le risque de contamination du contenu d'un récipient lors de l'installation de tels connecteurs.

Un autre objectif de la présente invention est de permettre à l'opérateur de vérifier très aisément l'intégrité d'une substance active contenue dans un ensemble connecteur/flacon pré-connecté avant sa connexion à une poche à soluté par exemple.

A cet effet, l'invention concerne un connecteur luer ayant un conduit interne pour le passage d'un fluide.

Selon l'invention, ce connecteur luer comprend:
- des moyens de liaison à un second connecteur luer ayant un perforateur, ces moyens de liaison étant placés à une de ses extrémités,
- cette extrémité comprenant également un bourrelet continu formant saillie dans le conduit de manière à assurer l'étanchéité de ce conduit lors de l'introduction du perforateur du second connecteur luer dans ce conduit en vue de l'accouplement de ces connecteurs, et
- une membrane sécable est placée dans le conduit de manière à être séparée lorsque le second connecteur luer est accouplé au connecteur luer.

Le bourrelet continu constitue un joint d'étanchéité qui permet dès sa mise en contact avec le perforateur du second connecteur luer de former une liaison étanche entre ce bourrelet et la surface externe du perforateur.

Ainsi lorsque le perforateur est introduit plus avant dans le conduit pour réaliser l'accouplement des connecteurs luer mâle et femelle, la séparation de la membrane n'entraîne pas d'autre introduction d'air dans le flacon que celui déjà contenu dans le conduit. La liaison étanche forme une barrière au passage de l'air extérieur dans le flacon. Les risques d'une éventuelle contamination du contenu du flacon sont ainsi grandement minimisés.
Dans différents modes de réalisation particuliers de ce connecteur luer, chacun ayant ses avantages particuliers et susceptibles de nombreuses combinaisons techniques possibles:
- la membrane comporte une ou plusieurs zones prédéterminées d'affaiblissement pour faciliter sa séparation,
- la membrane comporte une zone de renfort sur une portion de son pourtour de sorte que ladite membrane reste solidaire du conduit interne du connecteur luer,
- la membrane est placée dans le conduit de manière à ce que le perforateur du second connecteur recouvre la ou les portions de la membrane séparée après accouplement des connecteurs,
   Que la membrane ait été séparée en un seul tenant du conduit ou en plusieurs portions par le perforateur, ce mode de réalisation permet avantageusement d'écarter cette membrane de la voie centrale de communication de fluide déterminée par le conduit interne du connecteur luer. On évite d'obstruer le conduit assurant la communication de fluide et on minimise ainsi les pertes éventuelles de principe actif après reconstitution.
- les moyens de liaison comprennent une virole ou un filetage externe,
- il comporte un organe de verrouillage destiné à coopérer avec un organe de verrouillage complémentaire placé sur ledit second connecteur pour bloquer en position accouplée lesdits connecteurs luer,
   Cet organe de verrouillage comporte de préférence des moyens de type cliquet anti-retour.
- l'extrémité du connecteur luer comprend de plus un élément sécable.

L'invention vise aussi un connecteur médical pour récipient comportant un bouchon perçable, ce connecteur comprenant un élément de couplage comportant une paroi annulaire entourant un élément de perçage.

Selon l'invention,
- l'élément de perçage comprend un conduit interne en communication de fluide avec une portion de tube, cette portion de tube étant placée au moins partiellement en saillie de l'élément de couplage,
- un connecteur luer tel que décrit précédemment est partiellement inséré dans la portion de tube, et
- le connecteur médical est monobloc.
Dans différents modes de réalisation particuliers de ce connecteur médical, chacun ayant ses avantages particuliers et susceptibles de nombreuses combinaisons techniques possibles:
- le connecteur luer ne comportant pas d'organe de verrouillage, le connecteur médical comporte un organe de verrouillage destiné à coopérer avec un organe de verrouillage complémentaire placé sur le second connecteur pour bloquer en position accouplée ces connecteurs luer,
- l'organe de verrouillage comporte des moyens de type cliquet anti-retour placés à l'extrémité de la portion de tube,
- la portion de tube ayant un diamètre interne d, le connecteur luer comporte un conduit interne de diamètre d₁ < d,

L'invention concerne également un set de transfert pour la préparation d'un mélange à des fins médicales.

Selon l'invention, ce set de transfert comprend un connecteur luer tel que décrit précédemment et un second connecteur luer comportant à une extrémité un perforateur et un organe de verrouillage complémentaire pour bloquer en position accouplée ces connecteurs luer.

L'invention vise enfin un set de transfert pour la préparation d'un mélange à des fins médicales.

Selon l'invention, ce set de transfert comprend un connecteur médical tel que décrit précédemment et un second connecteur luer comportant à une extrémité un perforateur et un organe de verrouillage complémentaire destiné à coopérer avec l'organe de verrouillage dudit connecteur médical pour bloquer en position accouplée ce second connecteur luer et ce connecteur médical.

De préférence, ce second connecteur luer comporte à son extrémité, une section sécable destinée à être engagée dans l'extrémité d'une tubulure.

Ce set de transfert permet avantageusement d'établir une pré-connexion entre un flacon contenant un principe actif et un récipient tel qu'une poche à soluté ou un flacon, contenant un constituant liquide.

Cette pré-connexion est réalisée de manière particulièrement sûre du fait du verrouillage de l'accouplement de chaque paire de connecteurs, et évite tout contact cutané ou aérien du personnel soignant avec des substances potentiellement très actives.

La connexion verrouillée entre le récipient contenant le constituant liquide et le flacon contenant le principe actif évite toute contamination également pendant les opérations d'élimination des poches après usage, puisque l'ensemble est éliminé sans séparation de ces éléments ainsi assemblés.

On a ainsi, par une manipulation simple, pratique et rapide, l'assurance de réaliser des mélanges sans perte d'un principe actif et d'un constituant liquide.

L'invention sera décrite plus en détail en référence aux dessins annexés dans lesquels:
- la figure 1 est une représentation schématique d'un connecteur médical selon un mode de réalisation de l'invention;
- la figure 2 est une vie en coupe selon l'axe A-A du connecteur de la Figure 1, l'élément sécable placé à l'extrémité du connecteur luer femelle ayant été ôté ;
- la figure 3 représente schématiquement une membrane comprenant une zone d'affaiblissement et une zone de renfort selon un premier mode de réalisation de l'invention;
- la figure 4 représente schématiquement des variantes de membrane comprenant des zones d'affaiblissement;
- la figure 5 montre schématiquement un set de transfert selon un mode de réalisation particulier de l'invention;

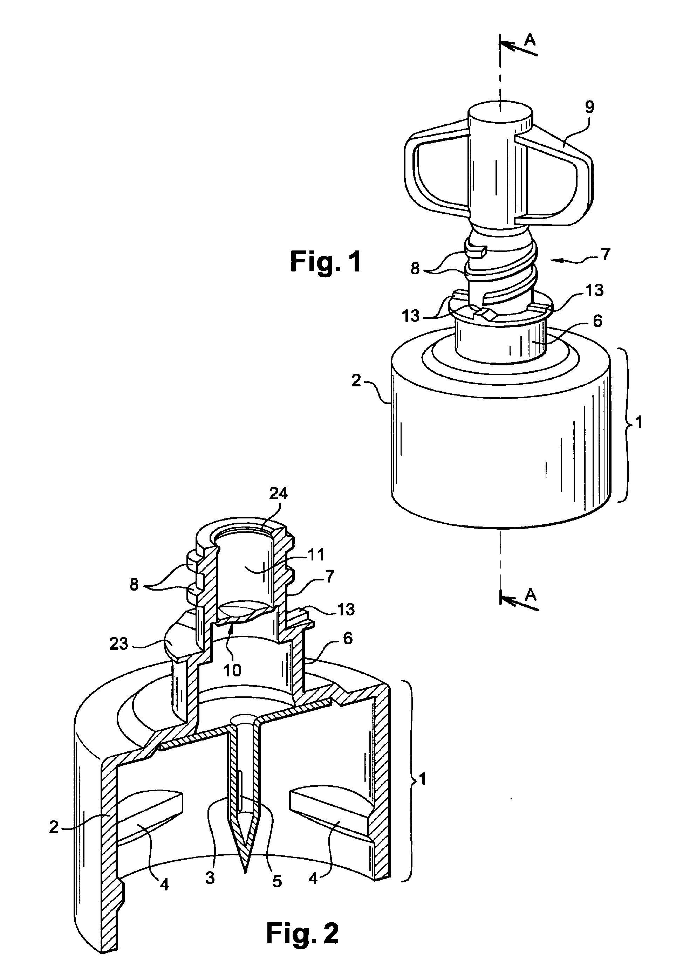

La Figure 1 montre un connecteur médical pour récipient comportant un bouchon perçable selon un mode de réalisation particulier de l'invention. Ce connecteur monobloc comprend un élément de couplage 1 comportant une paroi annulaire 2 entourant un élément de perçage 3 (Fig. 2).

La paroi annulaire 2 comporte un élément de fixation 4 dans sa partie inférieure, lequel est destiné à assurer la solidarisation de l'élément de couplage 1 avec l'extrémité du flacon sur lequel le connecteur médical est destiné à être connecté. La hauteur de la paroi annulaire 2 est, de préférence, inférieure à 15 mm ce qui rend ce connecteur médical adaptable sur tout type de flacon, qu'elle que soit sa forme.

Cet élément de fixation 4 est de préférence un bourrelet, continu ou non (Fig. 2), placé sur la surface intérieure de la paroi annulaire 2.

L'élément de perçage 3 qui est ici une protubérance pointue ("spike"), comprend un ou plusieurs orifices 5 à son extrémité ou à proximité de celle-ci. Ces orifices 5 communiquent avec le conduit interne de l'élément de perçage 3, lequel est en communication de fluide avec une portion de tube 6 faisant saillie de l'élément de couplage 1. Cette portion de tube 6 présente un diamètre interne d.

Dans le prolongement de cette portion de tube 6 est placé un connecteur luer femelle 7. Le corps de ce connecteur luer femelle 7 est légèrement inséré dans la portion de tube 6.

Ce connecteur luer femelle 7 comporte à son extrémité opposée à la portion de tube 6 un filetage externe 8 destiné à coopérer, de manière connue, avec une virole cylindrique d'accouplement d'un connecteur luer mâle du set de transfert.

Un élément sécable 9 est également solidaire de cette extrémité du connecteur luer femelle 7. Cet élément sécable 9 est avantageusement un bouchon quart de tour lié par un mince pontage à l'extrémité du corps du connecteur luer femelle 7. Ce bouchon quart de tour peut être désolidarisé de l'extrémité du connecteur luer femelle par une simple rotation manuelle d'un quart de tour.

Cet élément sécable 9 a un double rôle. Il permet tout d'abord à l'opérateur de vérifier très aisément, par un simple coup d'oeil, l'intégrité d'une substance active contenue dans un ensemble connecteur/flacon pré-connecté.

Mais il permet également de former une double barrière de protection du contenu du flacon avec une membrane 10 placée dans le conduit interne 11 du connecteur luer femelle 7.

Ainsi, le conduit interne 11 est protégé d'une éventuelle contamination tant que le bouchon quart de tour n'a pas été rompu et, en même temps, le contenu du flacon n'est à aucun moment exposé à l'air puisque la membrane 10 est placée dans le conduit interne de sorte qu'elle n'est rompue que lorsqu'un connecteur luer mâle est accouplé au connecteur luer femelle 7 pour procéder à la reconstitution.

Par ailleurs, l'extrémité du connecteur luer femelle comprend un bourrelet continu 24 formant saillie dans le conduit interne 11 de manière à assurer l'étanchéité de ce conduit lors de l'introduction du perforateur du connecteur luer mâle dans le conduit en vue de l'accouplement de ces connecteurs.

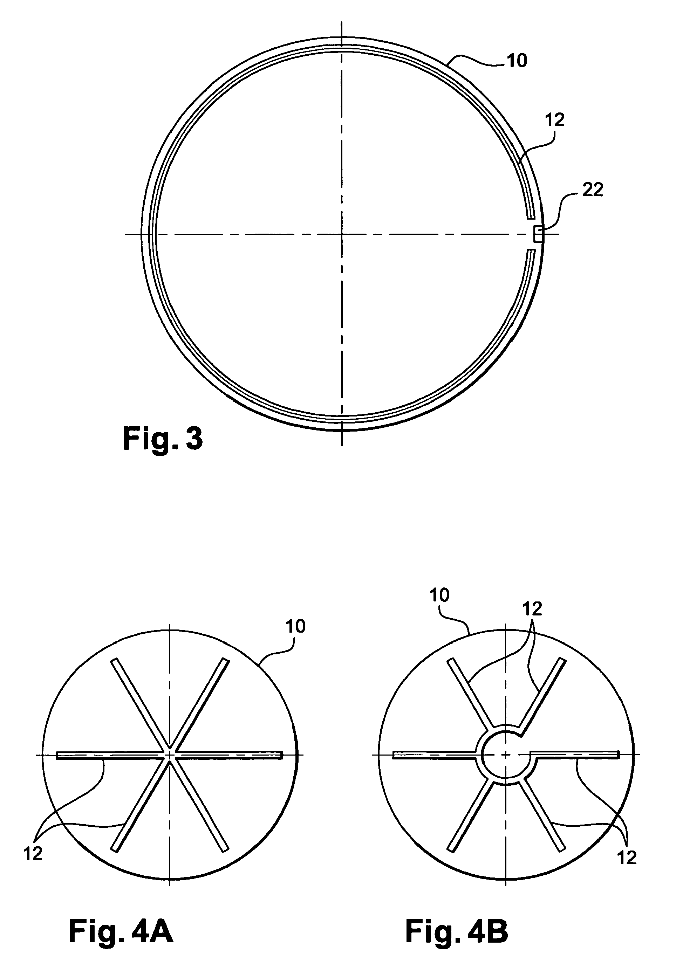

De préférence, la membrane 10 comporte une ou plusieurs zones prédéterminées d'affaiblissement 12 de manière à faciliter sa rupture, voire à provoquer la rupture de celle-ci en un nombre de morceaux prédéterminés. La Figure 3 montre une telle membrane 10 selon un mode de réalisation particulier. La zone d'affaiblissement 12 est destinée à provoquer une séparation en un seul tenant de la membrane du conduit interne du connecteur luer 7. La membrane 10 comporte également une zone de renfort 22 solidarisée au conduit interne de manière que cette membrane 10 reste liée au conduit interne 11 après séparation. Les Figures 4a) et 4b) montrent des variantes de membrane comprenant des zones d'affaiblissement 12 permettant une séparation en plusieurs portions de celle-ci.

Le conduit interne 11 du connecteur luer femelle 7 a un diamètre d₁ inférieur au diamètre d de la portion de tube 6. De plus, la membrane 10 est de préférence placée à une distance inférieure à son diamètre, qui est sensiblement égale à d₁, de l'extrémité opposée du connecteur luer femelle 7 de sorte qu'une partie au moins du ou des morceaux issus de la membrane séparée soient forcés hors du passage du fluide.

Le connecteur médical comporte également un organe de verrouillage 13 destiné à coopérer avec un organe de verrouillage complémentaire placé sur un connecteur luer mâle pour bloquer en position accouplée les connecteurs luer mâle et femelle 7.

Cet organe de verrouillage 13 comporte des moyens de type cliquet anti-retour. Ces moyens sont par exemple des marches inclinées ménagées circulairement sur la tranche d'une rondelle 23 coaxiale avec la portion de tube 6 et le connecteur luer femelle 7. Cette rondelle 23 est solidaire de l'extrémité de la portion de tube 6.

La membrane 10 est avantageusement conçue pour favoriser son injection lors de la fabrication du connecteur. De plus, cette membrane 10 ne génère pas de particules pouvant contaminer la solution médicamenteuse. Ce résultat est obtenu d'une part, en réalisant la membrane 10 avec des matériaux plastiques peu élastiques et d'autre part, en prévoyant des zones d'affaiblissement 12 très minces, par exemple de l'ordre du dixième de millimètre.

De même, la géométrie de l'élément de perçage 3 a été conçue de manière à perforer le bouchon du flacon sans générer de fragments. Cet élément de perçage 3 présente notamment peu ou pas d'arêtes vives.

Le connecteur médical est réalisé en un matériau plastique choisi de préférence dans le groupe comprenant le polycarbonate ou des dérivés de celui-ci, le polyoléfine (tel que le polyéthylène, le polypropylène, ou un copolymère de polypropylène) et le polystyrène. Il est de préférence réalisé par moulage.

L'invention concerne également un set de transfert pour la préparation d'un mélange à des fins médicales comprenant un connecteur médical tel que décrit précédemment.

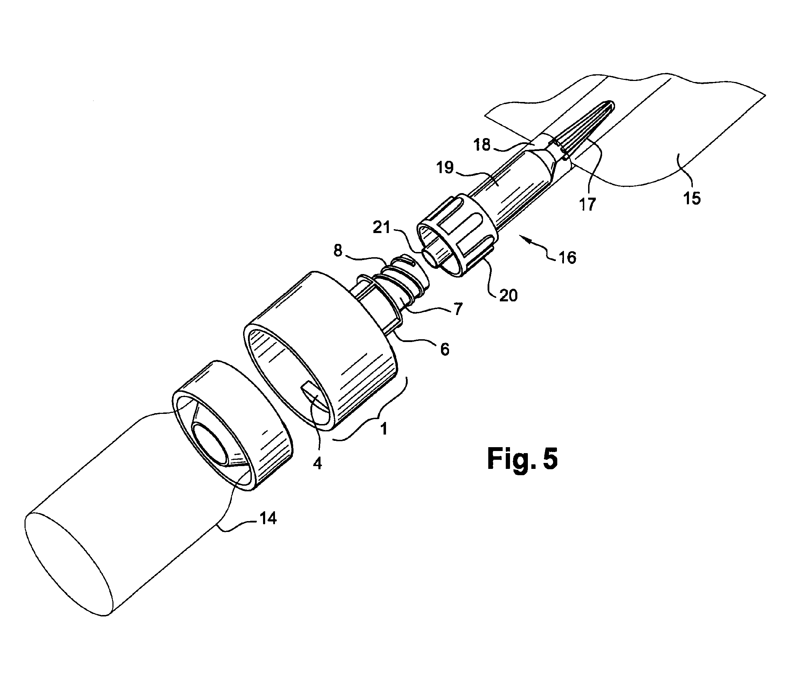

La Figure 5 montre un tel set de transfert selon un mode de réalisation de l'invention. Le connecteur médical est identique à celui décrit plus haut, et ne sera en conséquence pas décrit de nouveau. Ce connecteur est destiné à être connecté à un flacon 14 contenant une substance active que l'on cherche à mélanger avec un constituant liquide telle qu'un liquide à diluer contenu dans une poche souple 15 pour l'administrer à un patient.

Le set de transfert comporte un connecteur luer mâle 16. Ce connecteur luer mâle 16 comporte à une extrémité, une section sécable 17 engagée dans l'extrémité de la tubulure 18 solidaire de la poche souple 15. Le connecteur luer mâle 16 comprend un corps cylindrique 19 auquel est rattaché de manière connue la section sécable 17.

A son autre extrémité, le corps 19 du connecteur luer mâle 16 se prolonge par une virole cylindrique d'accouplement 20 munie intérieurement d'un filetage.

Le corps 16 est muni d'un conduit central se terminant, côté section sécable 17, au voisinage d'un pontage mince où se fera la séparation entre la section sécable 17 et le corps 16 du connecteur luer mâle 16, cependant qu'à son autre extrémité, le conduit central communique avec une partie tubulaire coaxiale 21 faisant légèrement saillie hors de la virole 20. Cette partie tubulaire 21 est encore connue sous le nom de "perforateur". Avantageusement, la paroi externe de la partie tubulaire 21 du connecteur luer mâle 16 est légèrement conique pour faciliter la séparation de la membrane 10.

Comme illustré par la figure 5, le connecteur luer mâle 16 est engagé à force dans l'extrémité de la tubulure 18. L'extrémité de cette tubulure peut par exemple, venir en butée contre la virole 20 qui présente un diamètre externe sensiblement supérieur à celui du corps principal 19 et correspondant sensiblement à celui du diamètre interne de la tubulure 18.

Le connecteur luer femelle 7 est susceptible de s'engager dans la virole 20 du connecteur luer mâle 16, la partie tubulaire 21, ou perforateur, pénétrant alors dans le conduit interne 11 du connecteur luer femelle 7.

Le filetage 8 placé sur la surface externe du corps du connecteur luer femelle 7 est destiné à coopérer avec le filetage de la virole 20, en sorte d'accoupler par vissage les connecteurs luer mâle 16 et femelle 7.

Le connecteur luer mâle 16 comporte un organe de verrouillage complémentaire, en regard de l'organe de verrouillage 13 du connecteur luer femelle 7. Cet organe de verrouillage comporte des marches inclinées complémentaires de celles de l'organe de verrouillage du connecteur femelle et ménagées circulairement sur la tranche de la virole 20.

Ces organes de verrouillage 13 constituent un dispositif de type cliquet et coopèrent pour, en fin de vissage, constituer un crantage anti-retour interdisant le dévissage des connecteurs luer mâle 16 et femelle 7 ainsi assemblés.

Le connecteur luer mâle est un connecteur monobloc obtenu directement par moulage.

## Revendications

1. Connecteur luer ayant un conduit interne (11) pour le passage d'un fluide, **caractérisé en ce qu'**il comprend :
- des moyens de liaison (8) à un second connecteur luer (16) ayant un perforateur (21), lesdits moyens de liaison étant placés à une de ses extrémités,
- ladite extrémité comprenant également un bourrelet continu (24) formant saillie dans ledit conduit (11) de manière à assurer l'étanchéité dudit conduit lors de l'introduction dudit perforateur dans ledit conduit (11) en vue de l'accouplement desdits connecteurs (7, 16), et
- une membrane étanche et sécable (10) est placée dans ledit conduit (11) de manière à être séparée lorsque ledit second connecteur luer (16) est accouplé audit connecteur luer (7).

2. Connecteur selon la revendication 1, **caractérisé en ce que** ladite membrane (10) est placée dans ledit conduit (11) de manière à ce que le perforateur (21) dudit second connecteur recouvre la ou les portions de la membrane (10) séparée après accouplement desdits connecteurs (7, 6).

3. Connecteur selon la revendication 1 ou 2, **caractérisé en ce que** ladite membrane (10) comporte une ou plusieurs zones prédéterminées d'affaiblissement (12) de ladite membrane.

4. Connecteur selon l'une quelconque des revendications 1 à 3, **caractérisé en ce que** lesdits moyens de liaison (8) comprennent une virole ou un filetage externe.

5. Connecteur selon l'une quelconque des revendications 1 à 4, **caractérisé en ce que** ladite extrémité du connecteur luer comprend de plus un élément sécable (9).

6. Connecteur selon la revendication 5, **caractérisé en ce que** ledit élément sécable (9) est un bouchon quart de tour.

7. Connecteur selon l'une quelconque des revendications 1 à 6, **caractérisé en ce qu'**il comporte un organe de verrouillage destiné à coopérer avec un organe de verrouillage complémentaire placé sur ledit second connecteur (16) pour bloquer en position accouplée lesdits connecteurs luer (7, 16).

8. Connecteur médical pour récipient comportant un bouchon perçable, ledit connecteur comprenant un élément de couplage (1) comportant une paroi annulaire (2) entourant un élément de perçage (3), **caractérisé en ce. que** :
- ledit élément de perçage (3) comprend un conduit interne en communication de fluide avec une portion de tube (6), ladite portion de tube (6) étant placée au moins partiellement en saillie dudit élément de couplage (1),
- un connecteur luer (7) selon l'une quelconque des revendication 1 à 6 étant partiellement inséré dans ladite portion de tube (6), et **en ce que**
- ledit connecteur médical est monobloc.

9. Connecteur selon la revendication 8, **caractérisé en ce qu'**il comporte un organe de verrouillage (13, 23) destiné à coopérer avec un organe de verrouillage complémentaire placé sur ledit second connecteur (16) pour bloquer en position accouplée lesdits connecteurs luer (7, 16).

10. Connecteur selon la revendication 9, **caractérisé en ce que** ledit organe de verrouillage (13, 23) comporte des moyens de type cliquet anti-retour placés à l'eXtrémité de ladite portion de tube (6).

11. Connecteur selon la revendication 10, **caractérisé en ce que** lesdits moyens de type cliquet anti-retour sont agencés sur une rondelle (23) solidaire de l'extrémité de ladite portion de tube (6).

12. Connecteur selon l'une, quelconque des revendications 8 à 11, **caractérisé en ce que** ladite portion de tube (6) ayant un diamètre interne d, ledit connecteur luer (7) comporte un conduit interne (11) de diamètre d₁ < d.

13. Connecteur selon l'une quelconque des revendications 8 à 12, **caractérisé en ce que** ladite paroi annulaire (2) comporte un élément de fixation (4) destiné à assurer la fixation dudit élément de couplage (1) avec l'extrémité dudit récipient et **en ce que** la hauteur de ladite paroi annulaire (2) est inférieure à 15 mm.

14. Set de transfert pour la préparation d'un mélange à des fins médicales, **caractérisé en ce qu'**il comprend un connecteur luer selon l'une quelconque des revendications 1 à 7, et un second connecteur luer (16) comportant à une extrémité un perforateur (21) et un organe de verrouillage complémentaire pour bloquer en position accouplée lesdits connecteurs luer (7, 16).

15. Set de transfert pour la préparation d'un mélange à des fins médicales, **caractérisé en ce qu'**il comprend un connecteur médical selon l'une quelconque des revendications 8 à 13, et un second connecteur luer (16) comportant à une extrémité un perforateur (21) et un organe de verrouillage complémentaire destiné à coopérer avec l'organe de verrouillage (13, 23) dudit connecteur médical pour bloquer en position accouplée ledit second connecteur luer (16) et ledit connecteur médical.

16. Set de transfert selon la revendication 14 ou 15, **caractérisé en ce que** ledit second connecteur luer (16) comporte à son extrémité, une section sécable (17) destinée à être engagée dans l'extrémité d'une tubulure (18).

## Claims

1. A Luer connector having an internal duct (11) for passage of a fluid, **characterised in that** it comprises:
- means (8) of connection to a second Luer connector (16) having a perforator (21), said connection means being placed at one of its ends,
- said end also comprising a continuous rim (24) projecting in said duct (11) so as to seal said duct when said perforator is introduced into said duct (11) with a view to coupling said connectors (7, 16), and
- a impervious breakable membrane (10) is placed in said duct (11) so as to be separated when said second Luer connector (16) is coupled to said Luer connector (7).

2. A connector according to claim 1, **characterised in that** said membrane (10) is placed in said duct (11) so that the perforator (21) of said second connector covers the portion or portions of the membrane (10) separated after coupling of said connectors (7, 16).

3. A connector according to claim 1 or 2, **characterised in that** said membrane (10) comprises one or more predetermined weakening zones (12) of said membrane.

4. A connector according to any one of claims 1 to 3, **characterised in that** said connection means (8) comprise a ferrule or an external thread.

5. A connector according to any one of claims 1 to 4, **characterised in that** said end of the Luer connector in addition comprises a breakable element (9).

6. A connector according to claim 5, **characterised in that** said breakable element (9) is a quarter-turn plug.

7. A connector according to any one of claims 1 to 6, **characterised in that** it comprises a locking member intended to cooperate with a complementary locking member placed on said second connector (16) in order to lock said Luer connectors (7, 16) in the coupled position.

8. A medical connector for a receptacle comprising a piercable plug, said connector comprising a coupling element (1) comprising an annular wall (2) surrounding a piercing element (3), **characterised in that**:
- said piercing element (3) comprises an internal duct in fluid communication with a tube portion (6), said tube portion (6) being placed at least partially projecting from said coupling element (1),
- a Luer connector (7) according to any one of claims 1 to 6 being partially inserted in said tube portion (6), and **in that**
- said medical connector is in a single piece.

9. A connector according to claim 8, **characterised in that** it comprises a locking member (13, 23) intended to cooperate with a complementary locking member placed on said second connector (16) in order to lock said Luer connectors (7, 16) in the coupled position.

10. A connector according to claim 9, **characterised in that** said locking member (13, 23) comprises means of the non-return catch type placed at the end of said tube portion (6) .

11. A connector according to claim 10, **characterised in that** said means of the non-return catch type are arranged on a washer (23) secured to the end of said tube portion (6).

12. A connector according to any one of claims 8 to 11, **characterised in that**, said tube portion (6) having an internal diameter d, said Luer connector (7) comprises an internal duct (11) of diameter d₁ < d.

13. A connector according to any one of claims 8 to 12, **characterised in that** said annular wall (2) comprises a fixing element (4) intended to fix said coupling element (1) with the end of said receptacle and **in that** the height of said annular wall (2) is less than 15 mm.

14. A transfer set for preparing a mixture for medical purposes, **characterised in that** it comprises a Luer connector according to any one of claims 1 to 7, and a second Luer connector (16) having at an end a perforator (21) and a complementary locking member for locking said Leur connectors (7, 16) in the coupled position.

15. A transfer set for preparing a mixture for medical purposes, **characterised in that** it comprises a medical connector according to any one of claims 8 to 13, and a second Luer connector (16) having at an end a perforator (21) and a complementary locking member intended to cooperate with the locking member (13, 23) of said medical connector in order to lock said second Luer connector (16) and said medical connector in the coupled position.

16. A transfer set according to claim 14 or 15, **characterised in that** said second Luer connector (16) has at its end a breakable section (17) intended to be engaged in the end of a tube (18).

## Patentansprüche

1. Luer-Verbinder, der eine innere Leitung (11) für den Durchgang eines Fluids besitzt, **dadurch gekennzeichnet, dass** er umfasst:
- Mittel (8) für die Verbindung mit einem zweiten Luer-Verbinder (16), der eine Lochungsvorrichtung (21) besitzt, wobei die Verbindungsmittel an einem seiner Enden angeordnet sind,
- wobei das Ende außerdem einen ununterbrochenen Wulst (24) aufweist, der in die Leitung (11) vorsteht, derart, dass die Dichtigkeit der Leitung bei der Einführung der Lochungsvorrichtung in die Leitung (11), um die Verbinder (7, 16) miteinander zu koppeln, sichergestellt ist, und
- wobei eine dichte und trennbare Membran (10) in der Leitung (11) angeordnet ist, derart, dass sie getrennt werden kann, wenn der zweite Luer-Verbinder (16) mit dem Luer-Verbinder (7) gekoppelt wird.

2. Verbinder nach Anspruch 1, **dadurch gekennzeichnet, dass** die Membran (10) in der Leitung (11) in der Weise angeordnet ist, dass die Lochungsvorrichtung (21) des zweiten Verbinders den oder die Abschnitte der getrennten Membran (10) nach der Kopplung der Verbinder (7, 16) abdeckt.

3. Verbinder nach Anspruch 1 oder 2, **dadurch gekennzeichnet, dass** die Membran (10) eine oder mehrere vorgegebene Schwächungszonen (12) der Membran aufweist.

4. Verbinder nach einem der Ansprüche 1 bis 3, **dadurch gekennzeichnet, dass** die Verbindungsmittel (8) einen Ringbeschlag oder ein Außengewinde aufweisen.

5. Verbinder nach einem der Ansprüche 1 bis 4, **dadurch gekennzeichnet, dass** das Ende des Luer-Verbinders außerdem ein trennbares Element (9) umfasst.

6. Verbinder nach Anspruch 5, **dadurch gekennzeichnet, dass** das trennbare Element (9) ein Viertelumdrehungsstopfen ist.

7. Verbinder nach einem der Ansprüche 1 bis 6, **dadurch gekennzeichnet, dass** er ein Verriegelungsorgan umfasst, das dazu bestimmt ist, mit einem komplementären Verriegelungsorgan zusammenzuwirken, das an dem zweiten Verbinder (16) angeordnet ist, um die Luer-Verbinder (7, 16) in der gekoppelten Position zu blockieren.

8. Medizinischer Verbinder für einen Aufnahmebehälter, der einen durchlochbaren Stopfen aufweist, wobei der Verbinder ein Kopplungselement (1) umfasst, das eine ein Durchlochungselement (3) umgebende Ringwand (2) enthält, **dadurch gekennzeichnet, dass**:
- das Durchlochungselement (3) eine innere Leitung umfasst, die mit einem Rohrabschnitt (6) in einer Fluidkommunikation steht, wobei der Rohrabschnitt (6) wenigstens teilweise von dem Kopplungselement (1) vorsteht,
- ein Luer-Verbinder (7) nach einem der Ansprüche 1 bis 6 teilweise in den Rohrabschnitt (6) eingesetzt ist und dass
- der medizinische Verbinder ein Monoblockverbinder ist.

9. Verbinder nach Anspruch 8, **dadurch gekennzeichnet, dass** er ein Verriegelungsorgan (13, 23) umfasst, das dazu bestimmt ist, mit einem komplementären Verriegelungsorgan zusammenzuwirken, das an dem zweiten Verbinder (16) angeordnet ist, um die Luer-Verbinder (7, 16) in der gekoppelten Position zu blockieren.

10. Verbinder nach Anspruch 9, **dadurch gekennzeichnet, dass** das Verriegelungsorgan (13, 23) Mittel des Rückkehrverhinderungs-Sperrklinkentyps ist, die am Ende des Rohrabschnitts (6) angeordnet sind.

11. Verbinder nach Anspruch 10, **dadurch gekennzeichnet, dass** die Mittel des Rückkehrverhinderungs-Sperrklinkentyps an einer Unterlegscheibe (23) angeordnet sind, die mit dem Ende des Rohrabschnitts (6) fest verbunden ist.

12. Verbinder nach einem der Ansprüche 8 bis 11, **dadurch gekennzeichnet, dass** dann, wenn der Rohrabschnitt (6) einen Innendurchmesser d besitzt, der Luer-Verbinder (7) ein inneres Rohr (11) mit einem Durchmesser d₁ < d umfasst.

13. Verbinder nach einem der Ansprüche 8 bis 12, **dadurch gekennzeichnet, dass** die Ringwand (2) ein Befestigungselement (4) umfasst, das dazu bestimmt ist, die Befestigung des Kopplungselements (1) an dem Ende des Aufnahmebehälters sicherzustellen, und dass die Höhe der Ringwand (2) kleiner als 15 mm ist.

14. Umladeanordnung für die Zubereitung eines Gemisches zu medizinischen Zwecken, **dadurch gekennzeichnet, dass** sie einen Luer-Verbinder nach einem der Ansprüche 1 bis 7 und einen zweiten Luer-Verbinder (16) umfasst, der an einem Ende eine Lochungsvorrichtung (21) und ein komplementäres Verriegelungsorgan, um die Luer-Verbinder (7, 16) in der gekoppelten Position zu blockieren, aufweist.

15. Umladeanordnung für die Zubereitung eines Gemisches zu medizinischen Zwecken, **dadurch gekennzeichnet, dass** sie einen medizinischen Verbinder nach einem der Ansprüche 8 bis 13 und einen zweiten Luer-Verbinder (16) umfasst, der an einem Ende eine Lochungsvorrichtung (21) und ein komplementäres Verriegelungsorgan aufweist, das dazu bestimmt ist, mit dem Verriegelungsorgan (13, 23) des medizinischen Verbinders zusammenzuwirken, um den zweiten Luer-Verbinder (16) und den medizinischen Verbinder in der gekoppelten Position zu blockieren.

16. Umladeanordnung nach Anspruch 14 oder 15, **dadurch gekennzeichnet, dass** der zweite Luer-Verbinder (16) an seinem Ende einen trennbaren Abschnitt (17) umfasst, der dazu bestimmt ist, mit dem Ende eines Rohrstutzens (18) in Eingriff zu gelangen.
